(19)

Europäisches
Patentamt

European
Patent Office

Office européen
des brevets

(11) **EP 2 916 829 B1**

(12) **EUROPEAN PATENT SPECIFICATION**

(45) Date of publication and mention
of the grant of the patent:
**27.02.2019 Bulletin 2019/09**

(21) Application number: **13776968.3**

(22) Date of filing: **14.10.2013**

(51) Int Cl.:
**A61K 9/50** *(2006.01)*

(86) International application number:
**PCT/EP2013/003089**

(87) International publication number:
**WO 2014/072015 (15.05.2014 Gazette 2014/20)**

(54) **USE OF SILICON OXIDE-BASED MATERIAL FOR THE MODIFIED RELEASE OF BIOLOGICALLY ACTIVE AGENTS**

VERWENDUNG VON AUF SILICIUMOXID BASIERENDEM MATERIAL FÜR DIE MODIFIZIERTE FREISETZUNG BIOLOGISCHER WIRKSTOFFE

UTILISATION DE MATÉRIAU À BASE D'OXYDE DE SILICIUM POUR LA LIBÉRATION MODIFIÉE D'AGENTS BIOLOGIQUEMENT ACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2012 EP 12007668**

(43) Date of publication of application:
**16.09.2015 Bulletin 2015/38**

(73) Proprietor: **Merck Patent GmbH
64293 Darmstadt (DE)**

(72) Inventors:
• **CABRERA, Karin
63303 Dreieich (DE)**
• **LANG, Ulrich
64646 Heppenheim (DE)**
• **PETERS, Benjamin
64839 Muenster (DE)**
• **SAAL, Christoph
64853 Otzberg (DE)**
• **SCHULZ, Michael
64285 Darmstadt (DE)**
• **LUBDA, Dieter
64625 Bensheim (DE)**
• **KUCERA, Shawn
Cedar Park, TX 78613 (US)**

(56) References cited:
**EP-A1- 2 110 414         WO-A1-2012/051341
US-A1- 2011 123 601     US-B1- 6 207 098**

• **DOADRIO A L ET AL: "A rational explanation of the vancomycin release from SBA-15 and its derivative by molecular modelling", MICROPOROUS AND MESOPOROUS MATERIALS, ELSEVIER SCIENCE PUBLISHING, NEW YORK, US, vol. 132, no. 3, 1 August 2010 (2010-08-01), pages 559-566, XP027049603, ISSN: 1387-1811 [retrieved on 2010-04-10] cited in the application**
• **ZHAO D ET AL: "NONIONIC TRIBLOCK AND STAR DIBLOCK COPOLYMER AND OLIGOMERIC SURFACTANT SYNTHESES OF HIGHLY ORDERED, HYDROTHERMALLY STABLE, MESOPOROUS SILICA STRUCTURES", JOURNAL OF THE AMERICAN CHEMICAL SOCIETY, ACS PUBLICATIONS, US, vol. 120, 1 January 1998 (1998-01-01), pages 6024-6036, XP002923902, ISSN: 0002-7863, DOI: 10.1021/JA974025I cited in the application**

**Description**

In recent years ordered porous materials have been increasingly studied for the use as drug delivery systems. From such materials mesoporous silica has been of specific interest.

One main approach for using mesoporous silica for the formulation of drug delivery systems is to increase the dissolution rate of poorly water-soluble or water-insoluble active pharmaceutical ingredients. Poorly water-soluble or insoluble active pharmaceutical ingredients usually have a very low bioavailability due to their poor solubility in gastrointestinal fluids such as gastric fluid and intestinal fluid causing incomplete absorption. The rationale of using mesoporous silica for use in drug delivery systems is to increase the dissolution rate of poorly water-soluble or water-insoluble active pharmaceutical ingredients and thereby to improve their bioavailability. Furthermore, mesoporous silica can be used to generate supersaturation of the active pharmaceutical ingredient with regards to the crystalline active pharmaceutical ingredient. Still further on, mesoporous silica can be used to stabilize this supersaturated state against precipitation of the active pharmaceutical ingredient.

Mesoporous materials, which have been extensively studied, are ordered silica such as MCM-41 and SBA-15. These ordered materials show a structure in which mesopores of a uniform size are regularly arranged.

SBA-15 was first described by Zhao et al. and is the result of a templating procedure based on a hexagonal arrangement of amphiphilic block copolymers (D.Y. Zhao et al.: Triblock copolymer syntheses of mesoporous silica with periodic 50 to 300 angstrom pores, Science 279 (1998) 548-552). MCM-41 is obtained by the template action of long chain alkylammonium surfactant molecules (J.S. Beck et al.: A new family of mesoporous molecular sieves prepared with liquid-crystal templates, J. Am. Chem. Soc. 114 (1992) 10834-10843). Typically, the pore diameter varies between 2 and 6 nm for MCM-41 and between 4 and 13 nm for SBA-15. In addition to the well-defined mesopore system, SBA-15 has a complementary pore system comprised of micropores (pore size < 2 nm). These micropores are located in the walls between adjacent mesopores and do not bridge the wall; thus, they constitute dead end pores (J.S. Beck et al.: A new family of mesoporous molecular sieves prepared with liquid-crystal templates, J. Am. Chem. Soc. 114 (1992) 10834-10843).

Vallet-Regi et al. were one of the first to explore the drug release properties of these materials in an attempt to prolong the release of ibuprofen using MCM-41 as a carrier (M. Vallet-Regi et al.: A new property of MCM-41: drug delivery system, Chem. Mater. 13 (2001) 308-311). The release kinetics of drugs from mesoporous silica carriers is dependent on several material characteristics including pore size (P. Horcajada et al.: Influence of pore size of MCM-41 matrices on drug delivery rate, Microporous Mesoporous Mater. 68 (2004) 105-109), pore connectivity (J. Andersson et al.: Influences of material characteristics on ibuprofen drug loading and release profiles from ordered micro- and mesoporous silica matrices, Chem. Mater. 16 (2004) 4160-4167) and the chemical composition of the silica surface (B. Munoz et al.: MCM-41 organic modification as drug delivery rate regulator, Chem. Mater. 15 (2003) 500-503).

Ordered mesoporous silica has also been used for drug delivery after functionalization with hydrophobic groups.

Doadrio et al. describe decreased release kinetics of vancomycin from SBA-15 after functionalizing with octylmethoxysilane (C8), compared to non-functionalized material from 0.890 min-½ to 0.068 min-½ (A. L. Doadrio et al.: A rational explanation of the vancomycin release from SBA-15 and its derivatives by molecular modeling, Microporous Mesoporous Mater. 132 (2010) 559-566). Doadrio et al. further describes the release of erythromycin from SBA-15 functionalized with C8 and C18 and observed that the release rate decreases with increasing of population of hydrophobic -CH2- moieties (A. L. Doadrio et al.: functionalized of mesoporous materials with long and can change as strategy for controlling drug delivery pattern, J. Mater. Chem. 16 (2006) 462-466). Izquierdo-Barba et al. investigated the release of erythromycin and ibuprofen from ordered mesoporous material MCM-48 and LP-Ia3d having pore sizes of 3.6 and 5.7 nm being modified with C8 and C18 hydrocarbon chains and observed a decrease of delivery rates when the surface of the material is modified (I. Izquierdo-Barba et al.: Release evaluation of drugs from ordered three-dimensional silica structures, Eur J Pharm Sci 26 (2006) 365-373). Qu et al. showed decrease of release rate of Captopril with increasing degree of C1 silylation of MCM-41 (F. Qu et al.: Effective controlled release of captopril by silylation of mesoporous of MCM-41, ChemPhysChem. 7 (2006) 400-406).

Bögershausen et al. describe a drug-carrier system consisting of silica hybrid gels having organic groups as an alternative approach to polymeric systems where the drug molecule is embedded in ordered mesoporous systems. The drug persantin was in situ incorporated into the silica hybrid gels during sol-gel synthesis from tetraethyl orthosilicate (TEOS) and a monomer precursor providing methyl or propyl functionalization. Release experiments show inhibition of drug release with an increasing number of methyl or propyl functionalization (A. Bögershausen et al.: Drug release from self-assembled inorganic-organic hybrid gels and gated porosity detected by positron annihilation lifetime spectroscopy, Chem. Mater. 18 (2006) 664-672). The bimodal silica system is describing an alternative to ordered silica gel. However, pore building strongly depends on the drug properties so that the synthesis of the material has to be adapted and optimized for each drug separately, resulting in a low flexibility of such a system.

US 6207098 B1 discloses a process for producing silica having mesopores and macropores and its functionalization with different groups including the hydrophobic groups C18-, C8-alkyl, butyl, phenyl and the use of such material in chromatography. US 7648761 B2 discloses a silica clad moulding having mesopores and macropores and its coating with different polymers, including hydrophobic polymers, which is used in chromatography. None of these, or any latter patent, teaches the use of such materials for the delivery of biologically active agents.

Shi et al. describe the synthesis of mesoporous silica particles, which beside the mesopores further contain macropores, and their functionalization by covalent binding with octadecyl as well as the use of such material for liquid chromatographic applications (Z.G. Shi et al.: Synthesis and characterization of hierarchically porous silica microspheres with penetrable macropores and tunable mesopores, Micropor. Mesopor. Mater. 116 (2008) 701-704). To provide macroporosity in the particles a sol-gel transition and phase separation technique is combined with an emulsion method. Shi et al. does not teach the use of the octadecyl functionalized material for the delivery of biologically active agents.

It has been found that silica material provides a useful tool for the modified release of biologically active agents if it contains mesopores and macropores and it is functionalized by coating with a polymer to provide hydrophobic surface properties. Therefore, the present invention is directed to the use of composite silicon oxide-based material for the modified release of biologically active agents, wherein the composite silicon oxide-based material is a substantially amorphous silicon oxide material which comprises macropores and mesopores and which is coated with a polymer to provide hydrophobic surface properties as defined in claim 1. Within the meaning of the present application the term "coated" shall mean that the polymer is adhered to the silicon oxide-based material, whereby such adherence to the silicon oxide-based material is given with or without formation of covalent bonds. The silicon oxide-based material can be "coated" by using the coating processes as described hereinafter.

Advantageously such material

- is provided without using a biologically active agent in its synthesis and thereby is flexible with regard to its use for modified release for various biologically active agents;

- can be easily loaded with the biologically active agent by suitable techniques known in the art, as, for example, by its adsorption from a solution in a suitable solvent or by wetness impregnation;

- provides better accessibility of mesopores for loading with, and release of, a biologically active agent due to the presence of macropores;

- can be provided in different grades of mesoporosity and/or microporosity, different hydrocarbon moieties and grades thereof so that a multitude of different materials having different release properties can be offered.

The term "composite material" as used herein refers to a material made from two constituent materials with different chemical and physical properties which are arranged in a layered manner and remain separate and distinct in the finished structure. In such composite material one constituent material constitutes the basic material, which is covered in a layered manner by the other constituent material which has different chemical and physical properties compared to the basic material. The term composite material encompasses material, wherein the whole surface of the basic material is covered, but also material, wherein the surface of the basic constituent material is only partly covered by the other constituent material. Variation of the extent of the surface coating provides a useful tool for variation of its physical properties and thereby to adapt the modified release properties to the specific requirements of the biologically active agent.

The term "silicon oxide-based composite material" as used herein refers to a composite material containing silicon oxide material as a basic constituent material which is provided with a layer of another material. In suitable silicon oxide-based composite material the surface of the basic constituent material is covered by the different material to an extent from 0.1 to 10 $\mu$mol/m$^2$, preferably from 0.1 to 4 $\mu$mol/m$^2$, more preferably from 0.1 to 3 $\mu$mol/m$^2$.

The term "biologically active agent" as used herein refers to any agent capable of eliciting a response in a biological system such as, for example, living cell(s), tissue(s), organ(s), and being(s). Biologically active agents can include natural and/or synthetic agents. Preferably the biologically active agent is a therapeutic agent, i.e. an agent used in the treatment, cure, prevention, or diagnosis of a disease or used to otherwise enhance physical or mental well-being. Preferred examples of a biologically active agent are pharmaceutical drugs, vitamins or minerals. In terms of its activity the biologically active agent can be, for example an agent that acts to control or prevent infection or inflammation, enhance cell growth and tissue regeneration, control tumor growth, act as an analgesic, promote anti-cell attachment or enhance bone growth, among other functions. Other suitable biologically active agents can include anti-viral agents, hormones, antibodies, or therapeutic proteins. Still other biologically active agents include prodrugs, which are agents that are not biologically active when administered but upon administration to a subject are converted to biologically active agents through metabolism or some other mechanism.

The term "modified release" as used herein refers to the release of the biologically active agent from the delivery system or a portion thereof upon contact of the delivery system or portion thereof with a liquid medium is different to the release of the same biologically active agent from a conventional immediate release formulation, wherein the release is mainly controlled by the solubility of the biologically active agent in the liquid medium. Accordingly, modified release includes, but is not limited to, accelerated release (i.e. an increased dissolution rate), sustained-release, extended release, slow release, delayed release and the like.

The term "amorphous" as used herein refers to a material with no long range order, although almost all materials are structured to some degree, at least on the local scale. An alternate term that has been used to describe these materials is "X-ray indifferent". For example, the microstructure of silica gels consists of 10-25 nm particles of dense amorphous silica, with porosity resulting from voids between the particles. Since there is no long range order in these materials, the pore sizes tend to be distributed over a rather large range. This lack of order also manifests itself in the X-ray diffraction pattern, which shows no distinct peaks but only a broad halo (S. Petit, G. Coquerel: "The amorphous state" 259-286 R. Hilfiker "Polymorphism in the Pharmaceutical Industry", Wiley-VCH 2006.

The term "hydrophobic surface" as used herein refers to a surface that is not wettable by water, as evidenced by a contact angle with water of greater than or equal to 70°, more typically greater than or equal to 90°, wherein the contact angle with water is measured by a conventional image analysis method, that is, by disposing a droplet of water on the substrate at 25°C, photographing the droplet, and measuring the contact angle shown in the photographic image. Alternatively evidence for hydrophobicity of the surface can also be given by Dynamic Vapor Sorption measurements (DVS, S. M. Reutzel-Edens, A.W. Newman: "Physical Characterization of Hygroscopicity in Pharmaceutical Solids", 235-258 R. Hilfiker "Polymorphism in the Pharmaceutical Industry", Wiley-VCH 2006). For such a coated, hydrophobic material water vapor sorption at a defined relative humidity (e.g. in the range 10 - 90 % r.h. at 25 °C or 40 °C) will be lower compared to the respective uncoated material (e.g. a water vapor sorption of the uncoated material at 40 % r.h. and 25 °C of 1.5 % compared to a water vapor sorption of the coated material at 40 % r.h. and 25 °C of 1.0 % would proof that the coated material is more hydrophobic)

Surface coverage by coating with hydrophobic polymer is calculated as described by E. Calleri et al. using equation:

$$\text{Surface coverage } [\mu mol/m^2]= 1/SBET * 106/((1201{,}1*(a/Pc\text{-}M))$$

wherein

SBET is the specific surface based on BET surface area measurement a is the number of C-atoms in the silanization group

Pc is the carbon content measured by elemental analysis

M is the molar mass of the silanization group.

(E. Calleri et al.: Evaluation of a monolithic epoxy silica support for penicillin G acylase immobilization, Journal of Chromatography A, 1031 (2004) 93 - 100)

According to the invention composite silicon oxide-based material is used, wherein the macropores have a diameter > 100 nm and the mesopores have the pore size in the range from 2 to 100 nm, preferably in the range from 4 - 100 nm, more preferably in the range from 6 - 50 nm. Therefore, the invention is also directed to the use of composite silicon oxide-based material for the modified release of biologically active agents, wherein the macropores have a mean diameter > 100 nm and the mesopores have the pore size in the range from 2 to 100 nm, preferably in the range from 4-100 nm, more preferably in the range from 6 - 50 nm.

Macropores and mesopores are arranged in the material in such a manner that the macropores build up a framework structure throughout the material and the mesopores are located on the surface of such framework. Such arrangement allows good accessibility of the mesopores and provides a particularly suitable basis for the loading and release of the biologically active agent.

The composite oxide-based material used for the modified release of biologically active agents can be in the form of monoliths or particles.

The term "monolith" as used herein refers to a body built-up in one single unit, piece or object having a size larger than 1 mm and at most about 10 cm. It may have various shapes, such as rods or films or pieces thereof. For example it may be a monolithic rod with a diameter larger than 1 mm and a length larger than 1 mm, or slices thereof. Preferably the monolith is a rod with a diameter from larger than 1 mm to 50 mm and a length from 1 mm to 200 mm, particular preferably from 5 mm to 150mm, or slices thereof having a diameter from 1 mm to 50 mm and a length from 1mm to 50mm, particular preferably from 1 mm to 25 mm. Slices of rods can be prepared by suitable cutting techniques such as, for example, sawing.

The term "particles" as used herein refers to solid particulate material having a size of at most 1 mm. The particles may have regular shapes, such as spheres, or irregular shapes. Depending from the application purpose particles of different sizes may be used such as nanoparticulate material having a size ranging from 10 nm to 100 nm, especially from 50 nm to 100 nm, microparticulate material having a size ranging from 100 nm to 100 $\mu$m or macroparticulate material having a size ranging from 100 $\mu$m to 1 mm.

According to a preferred embodiment of the invention the composite silicon oxide-based material used for the modified release of biologically active agents is in the form of particles which have a mean diameter from 0.5 $\mu$m to 500 $\mu$m. Therefore, a further object of the present invention is directed to the use of composite silicon oxide-based material for the modified release of biologically active agents, wherein the particles have a mean diameter from 0.5 $\mu$m to 500 $\mu$m.

The mean particle diameters and particle size distributions are measured using a laser diffraction instrument "Mastersizer 2000" available from Malvern Instruments. The particles are suspended by ultra sonic treatment in an aqueous solution with some ethanol and a dispersing agent for better wetting prior to the measurement. The measurement is performed in water at room temperature at a particle concentration in dispersion where the light obscuration in the laser diffraction instrument is usually about 10-15%.

According to an appropriate embodiment of the invention the composite silicon oxide-based material is used for poorly water-soluble biologically active agents, whereby the term "poorly water-soluble" is understood to encompass any biologically active agent, which has a solubility in water below 10 mg/ml when measured at 25°C. Preferably the biologically active agent has a solubility in the range from about 0.0001 mg/ml to about 5 mg/ml, particular preferably a solubility < 1 mg/ml. Therefore, the invention is also directed to the use of the composite silicon-oxide material for the modified release of biologically active agents, wherein the biologically active agent has a water solubility < about 10 mg/ml, preferably from about 0.0001 mg/ml to about 5 mg/ml and more preferably < 1 mg/ml, each measured at 25°C.

A biologically active agent, which can be used in the present invention is preferably classifiable as belonging to Class II or Class IV of the Biopharmaceutical Classification System and preferably has a water-solubility below about 2.5 mg/ml, even between 0.0001 and 1 mg/ml (i.e. " very slightly soluble" as defined in the United States Pharmacopeia), even below 0.1 mg/ml (i.e. " practically insoluble " as defined in the United States Pharmacopeia), at room temperature and physiological pH. Non-limiting examples of such drugs include for instance chlorothiazide, hydrochlorothiazide, nimodipine, flufenamic acid, furosemide, mefenamic acid, bendroflumethiazide, benzthiazide, ethacrinic acid, nitrendipine, itraconazole, saperconazole, troglitazone, prazosin, atovaquone, danazol, glibenclamide, griseofulvin, ketoconazole, carbamazepine, sulfadiazine, florfenicol, acetohexamide, ajamaline, benzbromarone, benzyl benzoate, betamethasone, chloramphenicol, chlorpropamide, chlorthalidone, clofibrate, diazepam, dicumarol, digitoxin, ethotoin, glutethimide, hydrocortisone, hydroflumethiazide, hydroquinine, indomethacin, ibuprofen, ketoprofen, naproxen, khellin, nitrazepam, nitrofurantoin, novalgin, oxazepam, papaverine, phenylbutazone, phenytoin, prednisolone, prednisone, reserpine, spironolactone, sulfabenzamide, sulfadimethoxine, sulfamerazine, sulfamethazine, sulfamethoxypyridazine, succinylsulfathiazole, sulfamethizole, sulfamethoxazole (also in admixture with trimethoprim), sulfaphenazole, sulfathiazole, sulfisoxazole, sulpiride, testosterone and diaminopyrimidines. Suitable examples of diaminopyrimidines include, without limitation, 2,4-diamino-5-(3,4,5-trimethoxybenzyl) pyrimidine (known as trimethoprim), 2,4-diamino-5-(3,4-dimethoxybenzyl) pyrimidine (known as diaveridine), 2,4 diamino-5-(3,4,6-trimethoxybenzyl) pyrmidine, 2,4-diamino-5-(2-methyl-4,5-dimethoxybenzyl) pyrimidine (known as ormetoprim), 2,4-diamino-5-(3,4-dimethoxy-5-bromobenzyl) pyrimidine, and 2,4-diamino-5-(4-chloro-phenyl)-6-ethylpyrimidine (known as pyrimethamine). The above-mentioned drugs are known as belonging to Class II (poorly soluble, highly permeable) or Class IV (poorly soluble, poorly permeable) of the Biopharmaceutical Classification System according to G. Amidon et al. in Pharm. Res. (1995) 12:413-420. Therefore, the object of the present invention is also directed to the use of the composite silicon oxide-based material for the modified release of biologically active agents, and wherein the biologically active agent belongs to the Class II or Class IV of the Biopharmaceutical Classification System (BCS).

In terms of its activity the biologically active agent can be, for example, an agent that acts to control or prevent infection or inflammation, enhance cell growth and tissue regeneration, control tumor growth, act as an analgesic, promote anti-cell attachment or enhance bone growth, among other functions. Other suitable biologically active agents can include anti-viral agents, hormones, antibodies, or therapeutic proteins. Still other biologically active agents include prodrugs, which are agents that are not biologically active when administered but upon administration to a subject are converted to biologically active agents through metabolism or some other mechanism.

Suitable composite material which can be used in the present invention can be provided by coating the constituent material with organic polymers providing the hydrophobic surface properties to the silicon oxide based material.

Organic polymers which are suitable for coating of the silicon oxide-based materials are organic materials which can be applied to the silicon oxide-based material as oligomer and/or polymer or organic oligomers and/or monomers which are applied to the silicon oxide-based material by polymerisation or polycondensation. The organic polymers can be chemi- or physisorbed on the silicon oxide-based material.

Suitable organic polymers which can be used to prepare the silicon oxide-based composite material are, for example, polystyrenes, polymethacrylates, polysiloxanes and derivatives thereof or copolymers of two or more suitable compounds, such as, for example, a coating of tetraalkoxysilane and methyltrialkoxysilane. Preference is given to chemi- or physisorbed polystyrenes, physisorbed poly(meth)acrylates or poly(meth)acrylic acid derivatives, such as, for example, poly(methacrylate), poly(2-hydroxyethyl methacrylate), a copolymer of 2-hydroxyethyl methacrylate and ethyl methacrylate or poly(octadecyl methacrylate) and silanes, which are especially preferred.

Coating process which can be used to prepare the silicon oxide-based composite material can take place by

- polymerisation or polycondensation of physisorbed monomers and/or oligomers without formation of covalent bonds to the silicon oxide-based material,

- polymerisation or polycondensation of physisorbed monomers and/or oligomers with formation of covalent bonds to the silicon oxide-based material,

- immobilisation (physisorption) of prepolymers without formation of bonds to the silicon oxide-based material or

- chemisorption of prepolymers on the silicon oxide-based material.

A solution which is employed for the coating of the silicon oxide-based material accordingly comprises either organic prepolymers or monomers and/or oligomers. In addition, it typically comprises a suitable solvent and optional further constituents, such as, for example, free-radical initiators. It is referred to in accordance with the invention as coating solution.

Prepolymers here means that use is made of already oligomerised and/or polymerised compounds which, after introduction into the silicon oxide-based material, are not subjected to any further polymerisation reaction, i.e. are not cross-linked further with one another. Depending on the nature of the application, they are adsorbed onto the silicon oxide-based material (physisorption) or covalently bonded to the silicon oxide-based material (chemisorption).

By contrast, monomers and/or oligomers are compounds which are suitable for polymerisation or polycondensation and which are crosslinked or polymerised further by polymerisation of polycondensation after introduction into the silicon oxide-based material. Oligomers here are compounds which have already been generated in advance by crosslinking or polymerisation of monomers.

Processes for providing composite silicon oxide-based material by coating are known to the person skilled in the art and described, for example, in Handbuch der HPLC [Handbook of HPLC], Ed. K. K. Unger; GIT-Verlag (1989) and Porous Silica, K. K. Unger, Elsevier Scientific Publishing Company (1979).

One process for the coating of particles includes the application of a polymer solution or a solution of monomer and free-radical initiator. The solvent is subsequently removed.

According to a preferred embodiment the composite silicon-based material used for the modified release of biologically active agents is provided by reaction of the amorphous silicon oxide material with a silane compound capable of forming a covalent bond with a silanol group of the amorphous silicon oxide material.

Examples of the silane compound capable of forming a covalent bond by being reacted with a silanol group of the amorphous silicon oxide material include silazane, siloxane, or alkoxysilane, and partial hydrolysates of silazane, siloxane, or alkoxysilane, or oligomers such as a polymerized dimmer to pentamer of silazane, siloxane or cyclic-siloxane, alkoxysilane.

Examples of the silazane include hexamethyldisilazane and hexaethyldisilazane.

Examples of the siloxane include hexamethyldisiloxane, 1,3-dibutyltetramethyldisiloxane, 1,3-diphenyltetramethyldisiloxane, 1,3-divinyltetramethyldisiloxane, hexaethyldisiloxane and 3-glycidoxypropylpentamethyldisiloxane.

Examples of the alkoxysilane include, for example, trimethylmethoxysilane, trimethylethoxysilane, trimethylpropoxysilane, phenyldimethylmethoxysilane, chloropropyldimethylmethoxysilane, dimethyldimethoxysilane, methyltrimethoxysilane, tetramethoxysilane, tetraethoxysilane, tetrapropoxysilane, tetrabutoxysilane, ethyltrimethoxysilane, dimethyldiethoxysilane, propyltriethoxysilane, n-butyltrimethoxysilane, n-hexyltrimethoxysilane, n-octyltriethoxysilane, n-octylmethyldiethoxysilane, n-octadecyltrimethoxysilane, phenyltrimethoxysilane, phenylmethyldimethoxysilane, phenetyltrimethoxysilane, dodecyltrimethoxysilane, n-octadecyltriethoxysilane, phenyltrimethoxysilane, diphenyldimethoxysilane.

These silane compounds may be used individually or in combination of two or more types thereof. Silane compounds having reactive groups capable of bonding colloidal silica particles with the polymer while curing the curable composition of the present invention can enhance the properties of the cured article, so that such silane compounds are preferred.

According to a preferred embodiment of the invention the composite silicon oxide-based material used for the modified release of the biologically active agent is modified by reaction with a compound having the formula (I)

SiXnR1(3-n)R2            (I)

wherein

X       is a reactive group,

R1      is C1-C5 alkyl,

n       is 1, 2 or 3; and

R2      is unsubstituted or substituted alkyl or aryl.

Therefore, one preferred object of the present invention is directed to the use of composite silicon oxide-based material for the modified release of biologically active agents, which is modified by reaction with a compound having the formula (I)

SiXnR1 (3-n)R2            (I)

wherein

X       is a reactive group,

R1      is C1-C5 alkyl,

n       is 1, 2 or 3; and

R2      is unsubstituted or substituted alkyl or aryl.

X is can be C1-C3 alkoxy, preferably methoxy or ethoxy or a halogen such as F, CI, Br or J, preferably CI.
In R2 alkyl can be unbranched or branched alkyl having 1 to 20 C atoms, which optionally may be substituted by 1, 2, 3 or 4 OH, Diol, NH2, Epoxy and/or CN whereby unbranched alkyl is preferred. Examples of suitable alkyl are methyl, ethyl, n-propyl, n-butyl, n-pentyl, n-hexyl, n-octyl, n-decyl, n-dodecyl or n-octadecyl, whereby n-octyl and n-octadecyl are preferred.
Aryl can be phenyl or phenylalkyl such as, for example phenylmethyl, phenylethyl, phenylpropyl or phenylbutyl, whereby phenylbutyl is preferred.
According to a particularly preferred embodiment in the silane compound of formula (I) used for the modification independendly from each other

X       is methoxy, ethoxy or halogen,

R2 is n-octyl, n-octadecyl, methyl, ethyl, n-propyl, isopropyl, n-butyl, isobutyl, tert.-butyl or phenylbutyl.
Therefore, one particularly preferred object of the invention is directed to the use of composite silicon oxide-based material for the modified release of biologically active agents, which is modified with a silane compound of formula (I),
wherein independendly from each other

X       is methoxy, ethoxy or halogen,

R2 is n-octadecyl, n-octyl, methyl, ethyl, n-propyl, isopropyl, n-butyl, isobutyl, tert.-butyl or phenylbutyl.
A material which is particular suitable to be used as composite material in accordance of the present invention can be easily provided by a process comprising the following steps:

(a) dissolving a water-soluble polymer or another pore forming agent and a precursor for a matrix dissolving agent in a medium that promotes the hydrolysis of the metalorganic compound (see step b);

(b) mixing a metalorganic compound which contains hydrolyzable ligands to promote hydrolysis reaction;

(c) solidifying the mixture through a sol-gel transition, whereby a gel is prepared which has three dimensional interconnected phase domains with one rich in solvent and the other rich in inorganic component in which surface pores are contained;

(d) disintegrating the gel into particles;

(e) setting the matrix dissolving agent free from its precursor, whereby the matrix dissolving agent modifies the structure of said inorganic component;

(f) removing the solvent;

(g) coating the material obtained by step (f) with a polymer to provide hydrophobic surface properties.

Therefore the present invention is further directed to a process for producing particulate composite silicon oxide-based material, wherein the particulate material is a substantially amorphous silicon oxide material which comprises macropores and mesopores and is coated with a polymer to provide hydrophobic surface properties and wherein the process includes the steps of:

(a) dissolving a water-soluble polymer or another pore forming agent and a precursor for a matrix dissolving agent in a medium that promotes the hydrolysis of the metalorganic compound (see step b);

(b) mixing a metalorganic compound or a mixture of metalorganic compounds which contains hydrolyzable ligands to promote hydrolysis reaction;

(c) solidifying the mixture through a sol-gel transition, whereby a gel is prepared which has three dimensional interconnected phase domains one rich in solvent the other rich in inorganic component in which surface pores are contained;

(d) disintegrating the gel into particles;

(e) setting the matrix dissolving agent free from its precursor, whereby the matrix dissolving agent modifies the structure of said inorganic component;

(f) removing the solvent;

(g) coating the material obtained by step (f) with a polymer to provide hydrophobic surface properties.

According to a preferred embodiment of the invention step (g) is performed by reaction of the material obtained by step (f) with a silane compound. Therefore, the invention is also directed to a process, wherein step (g) is performed by reaction of the material obtained by step (f) with a silane compound.

Removing of the solvent in step (f) can be performed by physical separation such as decantation or filtration. Preferably the separated particles are further dried under atmospheric or reduced pressure (evaporation drying) and/or heat treatment.

According to an alternative embodiment of the process for producing particulate composite silicon oxide-based material the disintegration step (d) is omitted and a milling step is introduced between steps (f) and (g).

The milling step can be performed by using the usual milling processes known in the art such as, for example, by using a pin mill or ball mill.

The process of the invention leads to particulate material having a mean diameter from about 1 $\mu$m to about 2000 $\mu$m, preferably from about 1 $\mu$m to 1000 $\mu$m and more preferably from about 1 $\mu$m to 500 $\mu$m.

Advantageously steps (a) to (f) of the process can be performed in the same reaction vessel (one-pot reaction), whereas the process described in the prior art involves an emulsification step, which requires distribution of the solution containing the water-soluble polymer (polyethylene oxide) and the metal organic compound (TEOS) in a different vessel containing paraffin oil. Preferably the vessel used for the process is a closable one, which allows the formation saturated vapor pressure, so that the liberation of the matrix dissolving agent from its precursor can be performed in an easy and time efficient manner as described later on. Surprisingly, the process of the invention leads to particulate material having a uniform particle size distribution, which can be adjusted to the requirements by controlling the process conditions. Advantageously time-consuming and expensive size classification steps as well as the loss of material caused by such classification steps can be avoided.

The process of the invention is based on the classical sol-gel method as known in the art, which in principle is a gel formation of metalorganic compound by polymerization under suitable conditions. When a metalorganic compound having hydrolyzable ligands is hydrolyzed by mixing with an acidic aqueous solution of water-soluble polymer or some other pore-forming phase, the subsequent sol-gel reaction results in the formation of solidified gel in which the phase separated domains, one rich in solvent the other rich in silica (gel skeleton, matrix), exist. After the solidification of the solution, the gel is aged by setting free the matrix dissolving agent from the precursor. The matrix dissolving agent leads to dissolution and re-precipitation of the inner wall, resulting in the loss of smaller pores and an increase of larger pores thereby creating sharply distributed mesopores.

Prior to further solidification, the gel skeleton is disintegrated into particles. Such disintegrating step can be performed by stirring as, for example, with an agitator, a high shear mixer (e.g. Ultraturrax®) or by sonication. The particle sizes and distribution can be controlled over a broad range by adjusting the starting time of the disintegration and/or the agitation speed. Surprisingly it has been found that very uniform particle distributions can be obtained by controlling such parameters. Therefore, the process of the invention further offers a simple method to produce particles having a uniform size distribution so that successive classification steps as described in the prior art can be avoided.

Preferably, the gel skeleton is transferred and homogenized to a particulate material by using an agitator. Particle size and distribution can be controlled by selecting the conditions of the process, especially the time period from phase separation until stirring as well as by the speed and size of the agitator. In principle, particle formation having a small size and a narrow particle size distribution is promoted by short time periods from phase separation until stirring and by using an agitator having larger blades whereas increased particle sizes is promoted by increasing the time period from phase separation until stirring and using an agitator having a smaller blade.

Typical time periods from phase separation until stirring are in the range from 15 to 120 minutes. For example, if the time period from phase separation until stirring is 15 minutes, and an agitator with a large blade is used a material having a mean particle size of about 11 $\mu$m and a narrow particle size distribution (d10: 5 $\mu$m, d50: 11 $\mu$m, d90: 21 $\mu$m) is obtained, if the time period is about 120 minutes a mean particle size of about 200 $\mu$m and a broader particle size distribution (d10: 5 $\mu$m, d50: 216 $\mu$m, d90: 956 $\mu$m) is obtained.

The term "particle size" within this patent application means the external dimension of the primary particle. Particle sizes and particle size distributions are measured by using laser diffraction and reported as volume weighted undersize diameters. For irregularly shaped particles the reported diameter is the diameter of a volume equivalent sphere. The d50 thus represents the mean (volume weighted) sphere equivalent diameter and is in this patent application sometimes referred to as mean particle size.

In the process of the present invention a water-soluble polymer suitable to induce pore formation by a phase separation process or other pore forming agents are being used to control porosity of the material. The pore forming agents have considerable solubility in water and water-alcohol mixed solvents and have to be uniformly dissolved in the solvent mixture generated during the hydrolysis reaction of metalorganic compound containing hydrolyzable ligands. Pore forming agents which can be used as part of the pore forming phase in producing the porous material according to the invention are desired to have considerable solubility in water and water-alcohol mixed solvents. They have to be uniformly dissolved in the solvent mixture generated during the hydrolysis reaction of the metalorganic compound containing hydrolyzable ligands, such as, for example, silicon alkoxide.

Water-soluble polymers suitable to induce pore formation are, for example, polymeric salts such as poly(sodium styrenesulfonate) or poly(potassium styrenesulfonate), polymeric acids which may dissociate to become polyanion such as poly(acrylic acid), polymeric bases which may dissociate to become polycation such as poly(allylamine) or poly(ethyleneimine), non-ionic polymers having ether oxygen in the main chain such as poly(ethylene oxide), non-ionic polymers having lactone, or lactone-type (e.g. lactam), units in the side chain such as poly(vinylpyrrolidone) are suitable examples. Preferred polymers are non-ionic surfactants such as ether derivatives of polyoxyethylene, especially those containing an alkyl-, aryl-, alkylaryl- (e.g. an alkylphenyl), or arylalkyl (e.g. phenylalkyl) residue. Non-ionic surfactants possessing polyoxypropylene residues as a hydrophilic moiety, such as polyoxypropylene alkyl ethers can also be used. Preferred polyethylene oxide containing surfactants are those which are derivatized with a lipophilic alkyl group with 8 to 20 C atoms, or with a lipophilic aryl group which can be substituted with one or several alkyl groups, and which have 6 to 25 C-atoms in total. Examples of the latter group of polyethylene oxide containing surfactants are polyoxyethylene nonylphenyl ether, polyoxyethylene octylphenyl ether, or polyoxyethylene (1,1,3,3-tetramethylbutyl)-phenyl ether. However, these examples are not limitative.

The hydrophilic lipophilic balance (HLB) system can be used to facilitate to estimate the behavior of nonionic surfactants, and can be used as guideline for exchanging different non-ionic surfactants. The amount of non-ionic surfactant to be added varies, depending on the type of said non-ionic surfactant and also on the type and the amount of the metal alkoxide added, but may be from 1.0 to 10.0 g, preferably from 1.5 to 6.0 g, per 10 g of the metal alkoxide.

The non-ionic surfactant has the function of inducing both sol-gel conversion and phase separation at the same time. While being gelled, the reaction system is separated into a solvent-rich phase and a silica rich phase. According to a preferred embodiment of the invention is directed to the process as described, wherein the pore forming agent is the non-ionic surfactant. Usable surfactants also include cationic surfactants.

Metalorganic compound can be applied by hydrolyzing metal alkoxides, metal chlorides, metal salts or coordinated compounds. In this process an organic polymer is used, which is compatible with the solution of the metal alkoxide or its polymer, and which undergoes phase separation during the hydrolysis-polymerization step. This method comprises preparing a gel which has a solvent-rich phase capable of giving macropores of not smaller than about 100 nanometers in size, through sol-gel conversion in the presence of a pore forming agent, and finally drying and calcining the material. The porous inorganic materials produced by this process display connected open macropores. Examples of pore forming agents disclosed in these documents are: Adding lower alkyl alcohols, like methanol or ethanol, to the gelling mixture can also be used to modify the size of the macropores. In the present invention the sol-gel method is used to control the pore size of the porous inorganic material.

Metalorganic compounds having a hydrolysable ligand, like metal alkoxides, are used as starting materials with additions of appropriate chemical substances to result in the formation of characteristic phase-separated structure of which solvent-rich pore forming phase converts to the macropore of the dried gel material: Such starting materials and the conditions necessary to hydrolyse these starting materials are known in the art. Preferred metal alkoxides are silicon alkoxides, which may include, for example, tetramethoxysilane (TMOS), tetraethoxysilane (TEOS), methyltrimethoxysilane, ethyltrimethoxysilane and vinyl trimethoxysilane. However, these examples are not limitative. Other suitable metal alkoxides or other suitable metal compounds including mixtures of these compounds are known in the art.

The conditions are chosen so as to hydrolyze the metal compound having a hydrolyzable functional group and to cause polymerization of the metal compound. At the same time the, sol-gel transition of the reacting solution and the phase separation into a solvent rich phase and a phase rich in metal compound (skeleton phase) are induced. For silicon alkoxides, as a metal compound having a hydrolyzable functional group, the hydrolysis is done in an acidic medium. Diluted organic or inorganic acids are preferred in this case. Especially preferred is the use of acetic acid, hydrochloric acid or nitric acid using concentrations between 1 mmol/l and 2 mol/l. Other acidic media suitable to carry out the hydrolysis of silicon alkoxides are known in the art. Suitable reagents for the hydrolysis of other metalorganic compounds are known in the art as well.

The process of the present invention further includes a precursor, i.e. a precursor for a matrix dissolving agent, which is used to control the mesoporosity of the material. Use of a precursor allows its addition from the beginning so that the precursor is, and remains, dissolved during sol-gel transformation. The liberation of the matrix dissolving agent can be induced later on, for example by heating, which leads to liberation of the matrix dissolving agent by chemical decomposition (thermolysis). Matrix dissolving agents to be liberated from the precursor are basic substances such as ammonia. Precursors which can used in the present invention to liberate ammonia are, for example, urea and organic amides such as formamide, N-methylformamide, N,N,-dimethylformamide, acetamide, N-methylacetamide, and N,N-dimethylacetamide. Preferred precursors are compounds having an amido group or an alkyl amido group, especially preferred is urea. Accordingly, one embodiment of the invention is directed to the process for producing the inorganic particulate material, wherein said precursor of the matrix dissolving agent is a compound having an amido group or an alkylamido group, preferably urea.

The amount of the thermolyzable compound in the reaction system of the present invention may vary, depending on the type of said compound. Urea, for example, may be used in an amount from 0.1 to 3 g, preferably from 0.2 to 2 g, per 10 g of the reaction system (reaction system = sum of all ingredients). The heating temperature for the thermolysis of urea may fall between 60°C and 200°C. It is preferred that the thermolyzing step is executed in a closed container in order to make the vapor pressure of the thermolyzed product saturated and to rapidly make the solvent have a steady pH-value. After the thermolysis, the pH of the solvent is preferably from 8.0 to 11.0. The time after which the pore structure of the gel stays substantially unchanged under the processing conditions depends on the type of the precursor for the matrix dissolving agent and on the conditions applied (e.g. the temperature); when urea is used as the precursor for the matrix dissolving agent the necessary time typically is between 30 minutes (e.g. at 200 °C) and 30 days (e.g. at 60°C). Preferably the gel is treated with urea at 110°C for about 4 hours which leads to a mesoporous material with ca. 10-13 nm pore size.

After the aging step of the gel by its interaction with the matrix dissolving agent which has been set free from the precursor and prior to removing the solvent from the solidified gel, the partly solidified gel skeleton is disintegrated into particles by appropriate means, preferably by stirring with an agitator.

Therefore, a further preferred embodiment of the invention is directed to the process for producing the inorganic particulate material, wherein step (e) is executed by stirring with an agitator, a high shear mixer or by ultrasonics. Early after the phase separation the gel is still soft which would possibly allow the use of an ultra sonic treatment for the particle formation.

After step (f) part for the manufacture of the particulate composite materials according to the present invention include an optional rinsing step, e.g. with water, a drying step, and a calcining step. Typically drying is achieved at temperatures between 20 and 150°C; this step can be facilitated using an oven with air circulation or by applying reduced pressure.

In step (g) the particulate silicon oxide material obtained by the execution of steps (a) to (f) is converted to particulate composite silicon oxide-based material by reaction with a silane compound. Suitable silane compounds are those as described above. According to a preferred embodiment of the invention the silane compound is the compound of formula (I) as described above, whereby such compound of formula (I), which is described to be a particularly preferred embodiment, is particularly preferred too. Reaction conditions are known to the person skilled in the art and described, for example, in Handbuch der HPLC [Handbook of HPLC], Ed. K. K. Unger; GIT-Verlag (1989) and Porous Silica, K. K. Unger, Elsevier Scientific Publishing Company (1979). In the present invention the silanization step is done in pure octadecylmethyldimethoxysilane used as suspension media at 120°C.

According to a preferred embodiment of the process the particulate material obtained by step (f) is calcined prior to the execution of step (g). Therefore, the invention is further directed to such process for producing particulate composite silicon oxide-based material, wherein the particulate material obtained by step (f) is calcined prior to the execution of step (g).

Calcining is typically done at final temperatures between 400 and 900°C for one to several hours. The final temperature is reached using a temperature program, typically raising the temperature between 50 and 200°C per hour.

After calcining the silica material can be rehydroxylated to convert the surface siloxanes back to silanols. Rehydroxylation can be achieved by treatment with water, aqueous acid or aqueous base. For example, the silica material is treated with ammonia or tertbutylammonium hydroxide solution pH 9.5 for 48 h at 60° C.

The pore size of macropores is determined using mercury porosimetry. It is also possible to estimate the pore dimensions from scanning electron micrographs (SEM). The pore size of mesopores and their specific surface area are determined using nitrogen adsorption/ desorption measurements (BET-method) which are performed by following standard protocols.

The silica particles having mesopores and macropores and which are functionalized by covalent bonding with octadecyl as described in the prior art are prepared by using an emulsion technique (Z.G. Shi et al. (2008), see above), whereas the particles obtained by the process of the present invention are formed by the disintegration of a gel after phase separation. Emulsification leads to spherical droplets of the gel, which are isolated and dried, which results in a particular material having a spherical shape. The process of the present invention does not use emulsification for particle formation but disintegration, and, therefore, leads to a new particulate product, which differs from the prior art product at least in that it has a different particle shape. Accordingly, the invention is also directed to a new inorganic particulate material mainly composed of silicon oxide, wherein the particulate material comprises macropores and mesopores, wherein the macropores have a mean diameter > 0.1 $\mu$m and the mesopores have a mean diameter between 2 and 100 nm, obtainable by the process of the present invention as described herein.

The particulate material obtainable by the process has a mean diameter from about 1 $\mu$m to about 2000 $\mu$m, preferably from about 1 $\mu$m to 1000 $\mu$m and more preferably from about 1 $\mu$m to 500 $\mu$m.

Further, one preferred embodiment of the invention is directed to such mesoporous particulate material, wherein said material has an irregular non-spherical shape.

According to the invention the composite silicon oxide-based material, including the material obtained by the process described above is used for the modified release of biologically active agents. The biologically active agent can be applied to the composite silicon oxide-based material by using the loading techniques known in the art, as, for example, by adsorption from a solution of the biologically active agent in a suitable solvent to the inorganic material and subsequent separation, by wetness impregnation of the inorganic material with a concentrated solution of the biologically active agent in a suitable solvent such as, for example, ethanol, $CH_2Cl_2$ or acetone and subsequent solvent evaporation, by spray-drying of a mixture of biologically active agent in a suitable solvent, by heating of a mixture of the biologically active agent and the particulate material, by drug loading with supercritical fluids or by sublimation of the biological active agent.

If loaded with a biologically active agent, the composite silicon oxide-based material constitutes a matrix for the biologically active agent from which upon contact with a liquid medium the biologically active agent is released in a different manner compared to a conventional immediate release formulation. Therefore, the present invention is also directed to a modified release delivery system comprising a biologically active agent and the composite silicon oxide-based material.

The composite silicon oxide-based material comprising macropores and mesopores as it is obtainable by the process of the present invention is especially usable to increase the dissolution rate of biologically active agents and to reach supersaturated states with regards to the thermodynamic solubility of the crystalline biologically active agent and further to decrease subsequent recrystallization, especially suitable to increase the dissolution rate and to decrease recrystallization of poorly water-soluble or water-insoluble biologically active agents. Poorly water-soluble substances are understood to have a solubility of 10 mg/ml, in particular < 5 mg/ml and more particularly < 1 mg/ml, practically water-insoluble or insoluble substances are those having a solubility in water of < 0.1 mg/ml.

Accordingly one further object of the present invention is directed to a modified release system comprising a biologically active agent and inorganic mesoporous and macroporous particulate material as it is obtainable by the process according to the present invention, wherein the biologically active agent has a solubility of < about 10 mg/ml, preferably from about 0.0001 mg/ml to about 5 mg/ml and more preferably from about < 1 mg/ml.

The modified release system according to the present invention contains the biologically active agent in an amount of from about 0.1 to about 90% by weight, preferably from about 0.2 to about 75% by weight, more preferably from about 5 to about 40% by weight most preferably from about 10 to about 30% by weight. Thus the invention is also directed to a modified release system as described herein, wherein the biologically active agent is present in an amount of from about 0.1 to about 90% by weight, preferably from about 0.2 to about 75% by weight, more preferably from about 5 to about 40% by weight most preferably from about 10 to about 30% by weight.

The modified release system can be formulated as an oral, a topical or a parenteral administration form, preferably as an oral administration form. Consequently, the invention is further directed to the use of the modified release system as described herein, wherein said system is an oral or a topical or a parenteral administration form.

Suitable for oral administration forms include tablets, capsules, powders, dragees, suspensions; suitable topical administration forms include ointments, creams, suspensions, lotions, powders, solutions, pastes, gels, sprays, aerosols or oils.

If an oral administration form is used, tablets, capsules and powders are preferred, if a topical administration form is used, ointments, creams, suspensions and powders are preferred. Accordingly, the invention is also directed to a modified release system as described herein, wherein said release system is an oral application form, which is a tablet, a capsule, a powder, or a dragee, or a topical administration form, which is an ointment, a cream, a suspension or a powder.

The modified release system is suitable to be used for the administration of at least one biologically active agent to mammal, preferably to a human. Accordingly, the invention is also directed to the use of the modified release system as described herein for the administration of at least one biologically active agent to a mammal, preferably to a human.

The application forms described above are well known in the art. For example, if the modified release system is in the form of a tablet or capsule, the biologically active agent loaded inorganic material can be combined with an oral, non-toxic and pharmaceutically acceptable inert excipient, such as, for example, ethanol, glycerol, water and the like. Powders can be composed of the biologically active agent loaded inorganic material itself, which may be further comminuted, or can be prepared, for example, by mixing the biologically active agent loaded inorganic, which may have been comminuted, with one or more comminuted pharmaceutical excipients, such as, for example, an edible carbohydrate, such as, for example, starch or mannitol. A flavour, preservative, dispersant and dye may likewise be present.

Capsules can be produced by preparing a powder mixture as described above and filling shaped gelatine or cellulosic shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the active agent loaded in an inorganic, which may have been comminuted in a suitable manner, with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tabletting machine, giving lumps of nonuniform shape which are broken up to form granules. The granules can be lubricated by addition of stearic acid, a stearate salt, talc or mineral oil in order to prevent sticking to the tablet casting moulds. The lubricated mixture is then pressed to give tablets. The biologically active agent loaded inorganic material can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

For the treatment of external tissue, for example mouth and skin, the formulations are preferably applied as topical ointment or cream. In the case of formulation to give an ointment, the biologically active agent loaded inorganic material can be employed either with a paraffinic or a water-miscible cream base. Alternatively, the biologically active agent loaded inorganic material can be formulated to give a cream with an oil-in-water cream base or a water-in-oil base.

Further formulations adapted for topical application in the mouth encompass lozenges, pastilles and mouthwashes.

The examples explain the invention without being restricted thereto.

Example 1

In a three necked flask (equipped with an overhead stirrer with a small blade, 7.6 cm diameter) 30.45 g PEO and 27.00 g urea are dissolved in 300 mL of 0.01 M acetic acid and mixed at room temperature for 10 min. The solution is then cooled down to 5.0°C followed by the addition of 150 mL TMOS without stirring. The resulting mixture is then stirred for 30 min at 5.0°C and subsequently heated up to 30°C for another 20 minutes. The stirring is then stopped and a phase separation takes place (solution changes from transparent to a white colour). 15 min after the phase separation the semi solid silica gel is stirred with a speed of 450 rpm for 3.5 h and with 300 rpm over night. Afterwards the silica gel is poured into a pressure resistant glass bottle and aged in a steam autoclave for 4 h at 110°C. The solvent is exchanged over a glass suction filter in four steps: purified water, nitric-acid, purified water and water/ethanol (2:1). The silica is washed four times with about 200 mL of each solvent and filtered to dryness. The semi-dried silica gel is replaced into an evaporating dish which is covered by a paper filter followed by a drying step in an oven for 5 days at 40°C.

The dried gel is calcined for 4 h at 600°C with a heating rate of 50 K/h. The calcined gel is analysed by Hg-Intrusion and N2-Adsorption/ Desorption (BET-measurement). Further, the particle size distribution is measured by the Malvern laser diffraction method.

Particle measurement of this and all other Examples was performed using the following Instruments:

Hg-Intrusion: PoreMaster 60 from Quantachrome Instruments, 1900 Corporate Drive Boynton Beach, Florida 33426 USA;

BET: Accelerated Surface Area and Porosimetry System ASAP® 2420 from Micromeritics Instrument Corporation, 4356 Communications Drive, Norcross, GA 30093-2901, USA;

Malvern Mastersizer 2000 from Malvern Instruments Ltd, Enigma Business Park, Grovewood Road, Malvern, Worcestershire WR14 1XZ, United Kingdom.

| | |
|---|---|
| Macropore size: | 4.81 $\mu$m |
| Mesopore size: | 10.1 nm |
| Surface area: | 322 m2/g |
| Particle size distribution: | d10 = 6 $\mu$m, d50 = 22 $\mu$m, d90 = 92 $\mu$m |

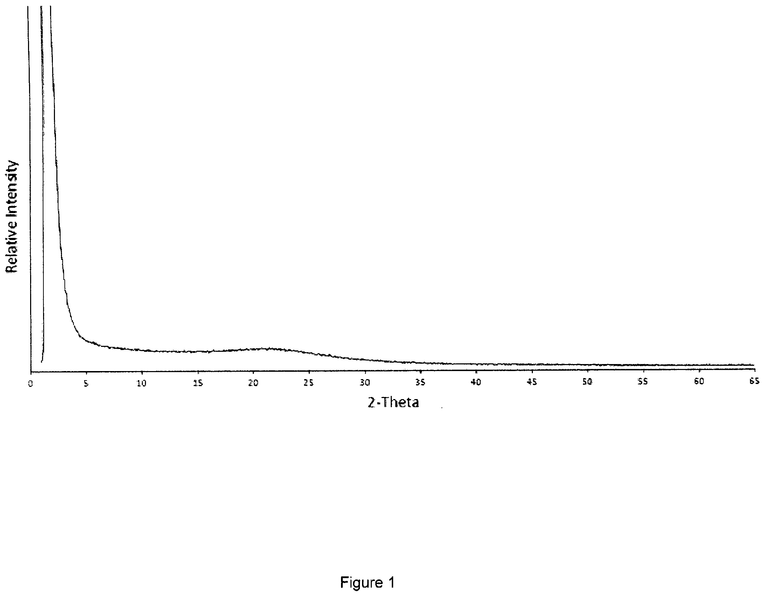

Amorphousness of the silica material was confirmed by Powder X-Ray Diffraction (PXRD) and Differential Scanning Calorimetry (DSC) analysis (PXRD data are shown in Figure 1, wherein the x axis shows the scattering angle 2-Theta (2$\theta$) and the y axis the relative intensity).

Example 2

In a three necked flask (equipped with an overhead stirrer with a large blade, 8.8 cm) 30.45 g PEO and 27.00 g urea are dissolved in 300 mL of 0.01 M acetic acid and mixed at room temperature for 10min. The solution is then cooled down to 5.0°C followed by the addition of 150 mL TMOS without stirring. The resulting mixture is then stirred for 30 min at 5.0°C and subsequently heated up to 30°C for another 20 minutes. The stirring is then stopped and a phase separation takes place (solution changes from transparent to a white colour). 15 min after the phase separation the semi solid silica gel is stirred with a speed of 450rpm for 3.5 h and with 300 rpm over night. Afterwards the silica gel is poured into a pressure resistant glass bottle and aged in a steam autoclave for 4 h at 110°C. The solvent is exchanged over a glass suction filter in four steps: purified water, nitric-acid, purified water and water/ethanol (2:1). The silica is washed four times with about 200 mL of each solvent and filtered to dryness. The semi-dried silica gel is replaced into an evaporating dish which is covered by a paper filter followed by a drying step in an oven for 5 days at 40°C.

The dried gel is calcined for 4 h at 600°C with a heating rate of 50 K/h. The calcined gel is analysed by Hg-Intrusion and N2-Adsorption/ Desorption (BET-measurements). Further, the particle size distribution is measured by the Malvern laser diffraction method.

| | |
|---|---|
| Macropore size: | 3.99 $\mu$m |

(continued)

| | |
|---|---|
| Mesopore size: | 10.2 nm |
| Surface area: | 321 m2/g |
| Particle size distribution: | d10 = 5 $\mu$m, d50 = 11 $\mu$m, d90 = 21 $\mu$m |

Example 3

In a three necked flask (equipped with an overhead stirrer with a large blade, 8.8 cm) 30.45 g PEO and 27.00 g urea are dissolved in 300mL of 0.01 M acetic acid and mixed at room temperature for 10 min. The solution is then cooled down to 5.0°C followed by the addition of 150 mL TMOS without stirring. The resulting mixture is then stirred for 30 min at 5.0°C and subsequently heated up to 30°C for another 20 minutes. The stirring is then stopped and a phase separation takes place (solution changes from transparent to a white colour). 30 min after the phase separation the semi solid silica gel is stirred with a speed of 450 rpm for 3.5 h and with 300 rpm over night. Afterwards the silica gel is poured into a pressure resistant glass bottle and aged in a steam autoclave for 4 h at 110°C. The solvent is exchanged over a glass suction filter in four steps: purified water, nitric-acid, purified water and water/ethanol (2:1). The silica is washed four times with about 200 mL of each solvent and filtered to dryness. The semi-dried silica gel is replaced into an evaporating dish which is covered by a paper filter followed by a drying step in an oven for 5 days at 40°C.
The dried gel is calcined for 4 h at 600°C with a heating rate of 50 K/h. The calcined gel is analysed by Hg-Intrusion and N2-Adsorption/ Desorption (BET-measurements). Further, the particle size distribution is measured by the Malvern laser diffraction method.

| | |
|---|---|
| Macropore size: | 1.7 $\mu$m |
| Mesopore size: | 10.1 nm |
| Surface area: | 321 m2/g |
| Particle size distribution: | d10 = 5 $\mu$m, d50 = 166 $\mu$m, d90 = 501 $\mu$m |

Example 4

In a three necked flask (equipped with an overhead stirrer with a large blade, 8.8 cm) 30.45 g PEO and 27.00 g urea are dissolved in 300 mL of 0.01 M acetic acid and mixed at room temperature for 10 min. The solution is then cooled down to 5.0°C followed by the addition of 150 mL TMOS without stirring. The resulting mixture is then stirred for 30min at 5.0°C and subsequently heated up to 30°C for another 20 minutes. The stirring is then stopped and a phase separation takes place (solution changes from transparent to a white colour). 30 min after the phase separation the semi solid silica gel is stirred with a speed of 450 rpm for 3.5 h and with 300 rpm over night. Afterwards the silica gel is poured into a pressure resistant glass bottle and aged in a steam autoclave for 4 h at 110°C. The solvent is exchanged over a glass suction filter in four steps: purified water, nitric-acid, purified water and water/ethanol (2:1). The silica is washed four times with about 200 mL of each solvent and filtered to dryness. The semi-dried silica gel is replaced into an evaporating dish which is covered by a paper filter followed by a drying step in an oven for 5 days at 40°C.
The dried gel is calcined for 4 h at 600°C with a heating rate of 50 K/h. The calcined gel is analysed by Hg-Intrusion and N2-Adsorption/ Desorption (BET-measurements). Further, the particle size distribution is measured by the Malvern laser diffraction method.
For the purpose of a subsequent rehydroxylation of the silica surface (transformation of siloxane groups to hydrophilic silanol groups) the calcined silica gel is suspended in a beaker with water which is placed in an autoclave for 3 h at 130°C. Afterwards the rehydroxylated gel is washed with methanol over a glass suction filter until all solvent is removed. The silica gel is then placed in an evaporating dish covered with a paper filter and dried in an oven for 5 days at 40°C.
The resulting material posess hydrophilic properties due to a maximization of silanol groups.

| | |
|---|---|
| Macropore size: | 1.43 $\mu$m |
| Mesopore size: | 11.1 nm |
| Surface area: | 328 m2/g |
| Particle size distribution: | d10 = 3 $\mu$m, d50 = 25 $\mu$m, d90 = 562 $\mu$m |

Example 5

507.5 g PEO and 450 g urea are added to an appropriate vessel, dissolved in 5L of 0.01 M acetic acid and mixed at room temperature for 10 min. The solution is poured into an agitator vessel with jacket (equipped with an overhead stirrer with a propeller mixer) and is then cooled down to 5.0°C followed by the addition of 2.5L TMOS without stirring. The resulting mixture is then stirred for 30min at 5.0°C and subsequently heated up to 30°C for another 20 minutes. The stirring is then stopped and a phase separation takes place (solution changes from transparent to a white colour). 15 min after the phase separation the semi solid silica gel is stirred with a speed of 300 rpm for 5min and within 10min the stirrer speed is increased up to 600rpm. The gel is stirred with a speed of 600rpm for 40min and with 300rpm over night. Afterwards the silica gel is aged in the vessel for 5 h at 80°C and then stirred at 30°C over night. The silica gel is poured into a glass suction filter and the solvent is exchanged in four steps: purified water, nitric-acid, purified water and water/ethanol (2:1). The silica gel is washed four times with about 8L of each solvent and filtered to dryness. The semi-dried silica gel is replaced into an evaporating dish which is covered by a paper filter followed by a drying step in an oven for 5 days at 40°C.

The dried gel is calcined for 4 h at 600°C with a heating rate of 50 K/h. The calcined gel is analysed by Hg-Intrusion and N2-Adsorption/ Desorption (BET-measurements). Further, the particle size distribution is measured by the Malvern laser diffraction method.

| | |
|---|---|
| Macropore size: | 5.12 $\mu$m |
| Mesopore size: | 4.3 nm |
| Surface area: | 698 m2/g |
| Particle size distribution: | d10 = 5 $\mu$m, d50 = 15 $\mu$m, d90 = 36 $\mu$m |

Example 6A (silanization of bimodal silica gel particles with C-18 groups):

In a three necked flask 50 g bimodal silica gel is suspended in 200 ml octadecylmethyldimethoxysilane. After suspending the bimodal silica gel the apparatus is heated up to 120°C with an oil bath and kept at 120°C for 24 h. After cooling down the reaction mixture to about 50°C a filtration is done followed by washing with petroleum, tetrahydrofuran and methanol. Finally the material is dried 4 hours at 100°C under vacuum.

Specific surface (before functionalisation): 698 m$^2$/g

Carbon content: 7.2%

Surface coverage: 0.5 $\mu$mol/M$^2$

Example 6B (silanization of bimodal silica gel particles with C-18 groups):

In a three necked flask 139 g bimodal silica gel is suspended in 600 ml octadecylmethyldimethoxysilane. After suspending the bimodal silica gel the apparatus is heated up to 120°C with an oil bath and kept at 120°C for 24 h. After cooling down the reaction mixture to about 50°C a filtration is done followed by washing with petroleum, tetrahydrofuran and methanol. Finally the material is dried 4 hours at 100°C under vacuum.

Specific surface (before functionalisation): 698 m$^2$/g

Carbon content: 9.1%

Surface coverage: 0.6 $\mu$mol/m$^2$

Example 6C: (silanization of bimodal silica gel with C-18 groups)

In a three necked flask 190 g bimodal silica gel is suspended in 900 ml Toluol. 394.9 g N,N-diethylaminoctadecyldimethylsilane is added. The apparatus is heated up to 120°C with an oil bath and kept at 120°C for 6h. After cooling down the reaction mixture filtration is done followed by washing with terahydrofuran and methanol. Finally the material is dried 4 hours at 100°C under vacuum.

Specific surface (before functionalisation): 678 m$^2$/g

Carbon content: 22.7 %

Surface coverage: 2.0 $\mu$mol/m$^2$

Example 7: (silanization of ordered silica gel MCM-41 with C-18 groups)

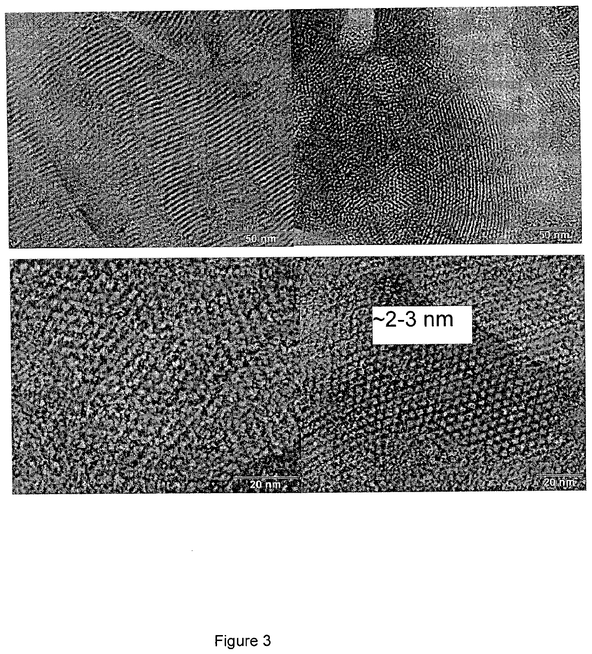

Prior silanization highly ordered mesoporous structure of MCM-41 material was confirmed by Transmission electron microscopy (TEM) (see Figure 3,).

In a three necked flask 40 g silica gel MCM-41 is suspended in 200 ml octadecylmethyldimethoxysilane. After suspending the silica gel MCM-41 the apparatus is heated up to 120°C with an oil bath and kept at 120°C for 24 h. After cooling down the reaction mixture to about 50°C filtration is done followed by washing with petroleum, tetrahydrofuran and methanol.

Finally the material is dried 4 hours at 100°C under vacuum.

Specific surface (before functionalisation): 910 m$^2$/g

Carbon content: 27 %

Surface coverage: 2.0 $\mu$mol/m$^2$

Example 8: (Drug loading)

Fenofibrate, a amphipathic carboxylic acid lipid lowering agent, which is poorly soluble in aqueous solutions (0.8 $\mu$g/mL; see S. Jamzad et al., AAPS PharmSciTech 7 (2006) E1-E6), was used as model drug.

The silica material of the present invention was drug loaded with fenofibrate by using wetness impregnation. For this purpose 1.0 g of fenofibrate was dissolved in 130 mL of acetone at 53°C. A 250 mL three necked flask (heated by a water bath at 60°C; equipped with an overhead stirrer and paddle) was filled with 2.3 g of silica material. The fenofibrate solution was added pro rata (10 mL per impregnation step) to the flask while acetone was evaporated by a nitrogen stream under stirring. The procedure of impregnating and subsequently evaporating was repeated until the entire fenofibrate solution was evaporated. Additionally, the obtained powder was dried under vacuum at 40°C over night. The resulting drug load aimed to 30% by weight.

Silica material of the Examples 5, 6A, 6B, 6C as well as of Example 7 with and without coating were loaded with fenofibrate in accordance with the procedure described above. The dissolution rates of the fenofibrate loaded silica material, a commercially available tablet containing micronized 48 mg fenofibrate and surfactants (Tricor®) and pure crystalline fenofibrate was tested using USP Apparatus II (rotating paddle) dissolution tester with on-line UV sampler and measurement system (conditions: simulated gastric fluid (SGF) without pepsin; 1000 mL vessel; 37°C; 75 rpm; 0.1% sodium dodecyl sulphate (SDS)).

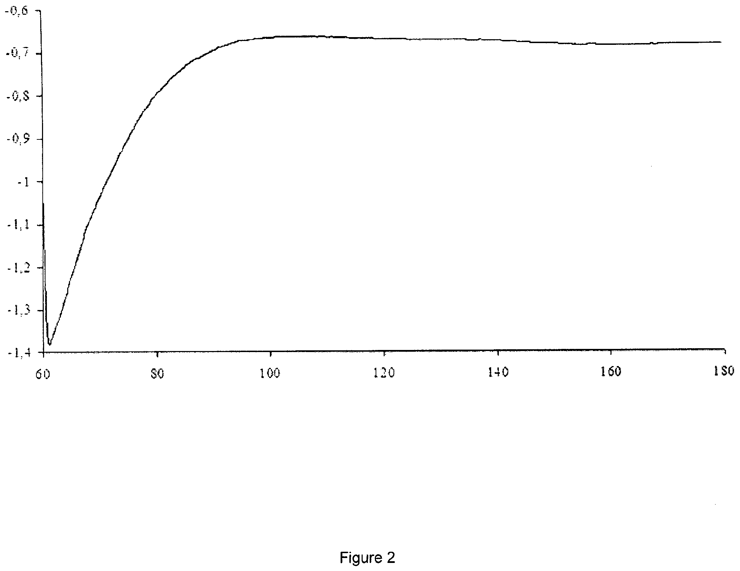

DSC measurement of drug-loaded silica material (Example 5) confirmed amorphousness of fenofibrate within such material. The data are shown in Fig. 2, wherein the x axis shows the temperature in decree Celsius and the y axis the shows the heat flow in Joule).

The fenofibrate loaded samples tested contained 50 mg of fenofibrate which was confirmed by high performance liquid chromatography (HPLC) with UV detector, pure crystalline fenofibrate was tested in the same amount (50 mg).

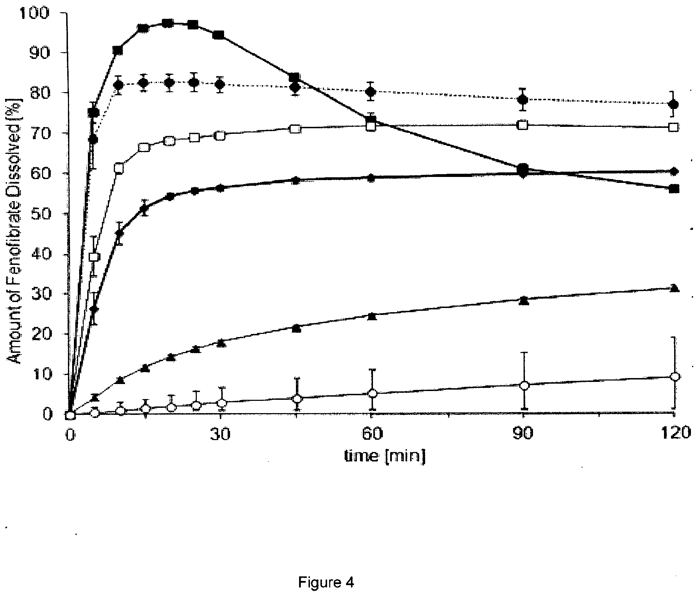

The dissolution rates of drug loaded samples with not coated material of example 5 (filled squares), coated material of examples 6A containing 7.2% carbon (open squares), 6B containing 9.1% (filled diamonds) and 6C containing 22.7% carbon (filled triangles), Tricor® (filled circles) and pure fenofibrate (open circles) are presented in Figure 4.

As apparent, dissolution rate of drug as well as maximum drug release decreases with increasing surface coating (as indicated by carbon content), The not coated material shows a rapid drug dissolution in the beginning till a high maximum above that of the coated materials but this is followed by a decrease below that of coated material 6A and 6B. All coated silica material does not show a decrease of dissolved drug due to recrystallization as is the case for the uncoated silica material as well as for the commercial reference product Tricor®. Therefore, in addition to the control of drug dissolution and its targeted controllability by variation of coating polymer the coated silica material provides a useful tool to avoid deviations in bioavailability caused by deviations in GIT passage times.

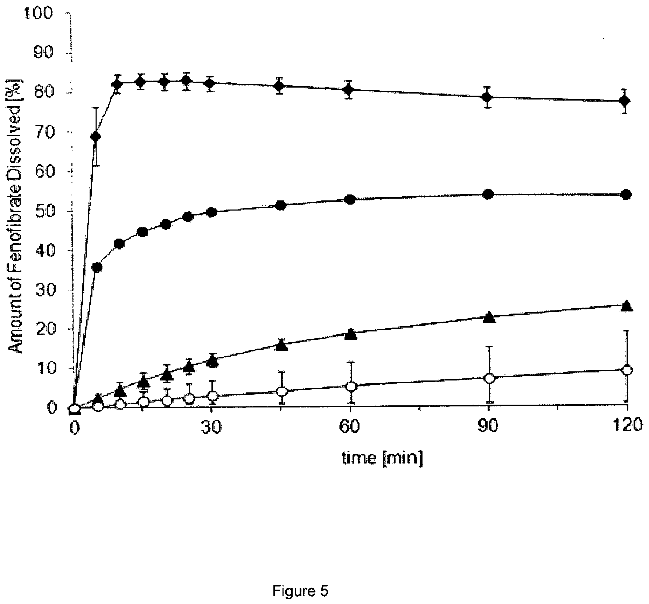

The dissolution rates of drug loaded samples of not coated material of example 7 (filled circles), coated material of example 7 containing 27% carbon (filled triangles), Tricor® (filled diamonds) and pure fenofibrate (open circles) are presented in Figure 5.

The data demonstrate the usefulness of the invention for the development of modified release drug formulations.

**Claims**

1. Use of composite silicon oxide-based material for the modified release of biologically active agents, wherein the composite silicon oxide-based material is a substantially amorphous silicon oxide material which comprises macropores and mesopores and which is coated with a polymer to provide hydrophobic surface properties, wherein the macropores have a mean diameter > 0.1 μm and the mesopores have a mean diameter from 2 to 100 nm.

2. Use of composite silicon oxide-based material according to Claim 1, wherein the composite material is in the form of monoliths or particles.

3. Use of composite silicon oxide-based material according to Claim 2, wherein the particles have a mean diameter from 0.5 μm to 500 μm.

4. Use of composite silicon oxide-based material according to one or more of Claims 1 to 3, wherein the biologically active agent has a water solubility < about 10 mg/ml, preferably from about 0.0001 mg/ml to about 5 mg/ml and more preferably from < 1 mg/ml, each measured at 25° C.

5. Use of composite silicon oxide-based material according to one or more of Claims 1 to 4, wherein the biologically active agent belongs to the Class II or Class IV of the Biopharmaceutical Classification System (BCS).

6. Use of composite silicon oxide-based material according to one or more of Claims 1 to 5, wherein the coating polymer is a silane compound having the formula (I)

$$SiX_nR1_{(3-n)}R2 \qquad (I)$$

wherein

X is a reactive group,
R1 is C1-C5 alkyl,
n is 1, 2 or 3; and
R2 is unsubstituted or substituted alkyl or aryl.

7. Use of composite silicon oxide-based material according to Claim 6, wherein in the silane compound of formula (I) independendly from each other

X is methoxy, ethoxy, propoxy, butoxy or halogen,
R2 n-octyl, n-octadecyl, methyl, ethyl, n-propyl, isopropyl, n-butyl, isobutyl, tert.-butyl or phenylbutyl

8. A process for producing particulate composite silicon oxide-based material, wherein the particulate material is a substantially amorphous silicon oxide material which comprises macropores and mesopores and is coated with a polymer to provide hydrophobic surface properties, wherein the macropores have a mean diameter > 0.1 μm and the mesopores have a mean diameter from 2 to 100 nm and wherein the process includes the steps of:

(a) dissolving a water-soluble polymer or another pore forming agent and a precursor for a matrix dissolving agent in a medium that promotes the hydrolysis of the metalorganic compound (see step b);
(b) mixing a metalorganic compound or a mixture of metalorganic compounds which contains hydrolyzable ligands to promote hydrolysis reaction;
(c) solidifying the mixture through a sol-gel transition, whereby a gel is prepared which has three dimensional interconnected phase domains one rich in solvent the other rich in inorganic component in which surface pores are contained;
(d) disintegrating the gel into particles;
(e) setting the matrix dissolving agent free from its precursor, whereby the matrix dissolving agent modifies the structure of said inorganic component;
(f) removing the solvent by evaporation drying and/or heat-treatment;
(g) coating the material obtained by step (f) with a polymer to provide hydrophobic surface properties.

9. Process according to Claim 8 wherein step (g) is performed by reaction of the material obtained by step (f) with a silane compound.

10. Process according to Claim 8 and/or 9, wherein the particulate material obtained by step (f) is calcined prior execution of step (g).

11. Composite silicon oxide-based material, wherein the composite silicon oxide-based material is in the form of particles and is substantially amorphous silicon oxide material which comprises macropores and mesopores and which is modified by reaction with silane compound to provide hydrophobic surface properties, wherein the macropores have a mean diameter > 0.1 $\mu$m and the mesopores have a mean diameter from 2 to 100 nm.

12. Composite silicon oxide-based material according to Claim 11, wherein the particles have an irregular non-spherical shape.

13. A modified release delivery system comprising a biologically active agent and composite silicon oxide-based material according to Claim 11 and/or 12.

14. A modified release delivery system according to Claim 13, wherein the biologically active agent is a pharmaceutical drug.

15. A modified release system according to Claim 13 and/or 14, wherein the biologically active agent has a water-solubility of < about 10 mg/ml, preferably from about 0.0001 mg/ml to about 5 mg/ml and more preferably from < 1 mg/ml.

16. A modified release system according to one or more of Claims 13 to 15, wherein the biologically active agent is present in an amount of from about 0.1 to about 90% by weight, preferably from about 0.2 to about 75% by weight, more preferably from about 5 to about 40% by weight most preferably from about 10 to about 30% by weight.

**Patentansprüche**

1. Verwendung von Verbundmaterial auf Siliziumoxidbasis zur modifizierten Freisetzung biologischer Wirkstoffe, wobei das Verbundmaterial auf Siliziumoxidbasis ein im Wesentlichen amorphes Siliziumoxidmaterial ist, das Makroporen und Mesoporen enthält und das zum Verleihen hydrophober Oberflächeneigenschaften mit einem Polymer beschichtet ist, wobei die Makroporen einen mittleren Durchmesser > 0,1 $\mu$m besitzen und die Mesoporen einen mittleren Durchmesser von 2 bis 100 nm besitzen.

2. Verwendung von Verbundmaterial auf Siliziumoxidbasis nach Anspruch 1, wobei das Verbundmaterial die Form von Monolithen oder Teilchen aufweist.

3. Verwendung von Verbundmaterial auf Siliziumoxidbasis nach Anspruch 2, wobei die Teilchen einen mittleren Durchmesser von 0,5 $\mu$m bis 500 $\mu$m besitzen.

4. Verwendung von Verbundmaterial auf Siliziumoxidbasis nach einem oder mehreren der Ansprüche 1 bis 3, wobei der biologische Wirkstoff eine Wasserlöslichkeit von < etwa 10 mg/ml, vorzugsweise von etwa 0,0001 mg/ml bis etwa 5 mg/ml und stärker bevorzugt von < 1 mg/ml, jeweils gemessen bei 25°C, aufweist.

5. Verwendung von Verbundmaterial auf Siliziumoxidbasis nach einem oder mehreren der Ansprüche 1 bis 4, wobei der biologische Wirkstoff zur Klasse II oder Klasse IV des biopharmazeutischen Klassifizierungssystems (BCS) gehört.

6. Verwendung von Verbundmaterial auf Siliziumoxidbasis nach einem oder mehreren der Ansprüche 1 bis 5, wobei das Beschichtungspolymer eine Silanverbindung der Formel (I) ist

$$SiX_nR1_{(3-n)}R2 \qquad (I),$$

worin

X eine reaktive Gruppe ist,
R1 für C1-C5-Alkyl steht,
n 1, 2 oder 3 ist und

R2 unsubstituiertes oder substituiertes Alkyl oder Aryl ist.

**7.** Verwendung von Verbundmaterial auf Siliziumoxidbasis nach Anspruch 6, wobei in der Silanverbindung der Formel (I) unabhängig voneinander
X Methoxy, Ethoxy, Propoxy, Butoxy oder Halogen ist,
R2 n-Octyl, n-Octadecyl, Methyl, Ethyl, n-Propyl, Isopropyl, n-Butyl, Isobutyl, tert.-Butyl oder Phenylbutyl.

**8.** Verfahren zur Herstellung von teilchenförmigem Verbundmaterial auf Siliziumoxidbasis, wobei das teilchenförmige Material ein im Wesentlichen amorphes Siliziumoxidmaterial ist, das Makroporen und Mesoporen enthält und zum Verleihen hydrophober Oberflächeneigenschaften mit einem Polymer beschichtet ist, wobei die Makroporen einen mittleren Durchmesser > 0,1 $\mu$m besitzen und die Mesoporen einen mittleren Durchmesser von 2 bis 100 nm besitzen, und wobei das Verfahren die folgenden Schritte umfasst:

(a) Lösen eines wasserlöslichen Polymers oder eines anderen porenbildenden Mittels und einer Vorstufe für ein Matrixlösungsmittel in einem Medium, das die Hydrolyse der metallorganischen Verbindung fördert (siehe Schritt b);
(b) Mischen einer metallorganischen Verbindung oder eines Gemischs von metallorganischen Verbindungen, die/das hydrolysierbare Liganden enthält, um die Hydrolysereaktion zu fördern;
(c) Verfestigen des Gemischs durch einen Sol-Gel-Übergang, wodurch ein Gel hergestellt wird, das dreidimensionale, miteinander verbundene Phasendomänen aufweist, von denen eine reich an Lösungsmittel, die andere reich an anorganischer Komponente ist, in der Oberflächenporen enthalten sind;
(d) Zerfallen des Gels in Teilchen;
(e) Freisetzen des Matrixlösungsmittels aus seiner Vorstufe, wodurch das Matrixlösungsmittel die Struktur der anorganischen Komponente modifiziert;
(f) Entfernen des Lösungsmittels durch Verdampfungstrocknung und/oder Wärmebehandlung;
(g) Beschichten des durch Schritt (f) erhaltenen Materials mit einem Polymer zum Verleihen hydrophober Oberflächeneigenschaften.

**9.** Verfahren nach Anspruch 8, wobei Schritt (g) durch Umsetzen des durch Schritt (f) erhaltenen Materials mit einer Silanverbindung durchgeführt wird.

**10.** Verfahren nach Anspruch 8 und/oder 9, wobei das durch Schritt (f) erhaltene teilchenförmige Material vor der Durchführung von Schritt (g) kalziniert wird.

**11.** Verbundmaterial auf Siliziumoxidbasis, wobei das Verbundmaterial auf Siliziumoxidbasis die Form von Teilchen hat und im Wesentlichen amorphes Siliziumoxidmaterial ist, das Makroporen und Mesoporen enthält und das durch Reaktion mit einer Silanverbindung zum Verleihen hydrophober Oberflächeneigenschaften modifiziert wird, wobei die Makroporen einen mittleren Durchmesser > 0,1 $\mu$m besitzen und die Mesoporen einen mittleren Durchmesser von 2 bis 100 nm besitzen.

**12.** Verbundmaterial auf Siliziumoxidbasis nach Anspruch 11, wobei die Teilchen eine unregelmäßige, nicht kugelförmige Gestalt haben.

**13.** Abgabesystem mit modifizierter Freisetzung, enthaltend einen biologischen Wirkstoff und ein Verbundmaterial auf Siliziumoxidbasis nach Anspruch 11 und/oder 12.

**14.** Abgabesystem mit modifizierter Freisetzung nach Anspruch 13, wobei der biologische Wirkstoff ein pharmazeutischer Arzneistoff ist.

**15.** System mit modifizierter Freisetzung nach Anspruch 13 und/oder 14, wobei der biologische Wirkstoff eine Wasserlöslichkeit von < etwa 10 mg/ml, vorzugsweise von etwa 0,0001 mg/ml bis etwa 5 mg/ml und stärker bevorzugt von < 1 mg/ml aufweist.

**16.** System mit modifizierter Freisetzung nach einem der Ansprüche 13 bis 15, wobei der biologische Wirkstoff in einer Menge von etwa 0,1 bis etwa 90 Gew.-%, vorzugsweise von etwa 0,2 bis etwa 75 Gew.-%, stärker bevorzugt von etwa 5 bis etwa 40 Gew.-%, am stärksten bevorzugt von etwa 10 bis etwa 30 Gew.-% vorliegt.

**Revendications**

1. Utilisation d'un matériau à base d'oxyde de silicium composite destiné à la libération modifiée d'agents biologiquement actifs, **caractérisée en ce que** le matériau à base d'oxyde de silicium composite est constitué d'un matériau à base d'oxyde de silicium essentiellement amorphe, qui comprend des macropores et des mésopores et qui est revêtu d'un polymère afin de fournir des propriétés de surface hydrophobes, où les macropores possèdent un diamètre moyen > 0,1 $\mu$m et les mésopores possèdent un diamètre moyen allant de 2 à 100 nm.

2. Utilisation d'un matériau à base d'oxyde de silicium composite selon la revendication 1, **caractérisée en ce que** le matériau composite se trouve sous la forme de monolithes ou de particules.

3. Utilisation d'un matériau à base d'oxyde de silicium composite selon la revendication 2, **caractérisée en ce que** les particules possèdent un diamètre moyen allant de 0,5 $\mu$m à 500 $\mu$m.

4. Utilisation d'un matériau à base d'oxyde de silicium composite selon l'une ou plusieurs parmi les revendications 1 à 3, **caractérisée en ce que** l'agent biologiquement actif possède une solubilité dans l'eau < environ 10 mg/ml, préférablement allant d'environ 0,0001 mg/ml à environ 5 mg/ml et plus préférablement < 1 mg/ml, chacune mesurée à 25°C.

5. Utilisation d'un matériau à base d'oxyde de silicium composite selon l'une ou plusieurs parmi les revendications 1 à 4, **caractérisée en ce que** l'agent biologiquement actif appartient à la Classe II ou la Classe IV du Système de Classification Biopharmaceutique (BCS).

6. Utilisation d'un matériau à base d'oxyde de silicium composite selon l'une ou plusieurs parmi les revendications 1 à 5, **caractérisée en ce que** le polymère de revêtement est un composé de silane répondant à la formule (I)

$$SiXnR1(3-n)R2 \qquad (I)$$

où

X est un groupement réactif,
R1 est $C_1$-$C_5$-alkyle,
n vaut 1, 2 ou 3 ; et
R2 est alkyle ou aryle non substitué ou substitué.

7. Utilisation d'un matériau à base d'oxyde de silicium composite selon la revendication 6, **caractérisée en ce que**, dans le composé de silane de formule (I), indépendamment les uns des autres,
X est méthoxy, éthoxy, propoxy, butoxy ou halogène,
R2 est n-octyle, n-octadécyle, méthyle, éthyle, n-propyle, isopropyle, n-butyle, isobutyle, tertio-butyle ou phénylbutyle.

8. Procédé de production d'un matériau à base d'oxyde de silicium composite particulaire, **caractérisé en ce que** le matériau particulaire est constitué d'un matériau à base d'oxyde de silicium essentiellement amorphe, qui comprend des macropores et des mésopores et qui est revêtu d'un polymère afin de fournir des propriétés de surface hydrophobes, où les macropores possèdent un diamètre moyen > 0,1 $\mu$m et les mésopores possèdent un diamètre moyen allant de 2 à 100 nm, et où le procédé comporte les étapes consistant à :

(a) solubiliser un polymère hydrosoluble ou un autre agent formateur de pores et un précurseur d'un agent de dissolution de matrice dans un milieu qui favorise l'hydrolyse du composé métallo-organique (voir étape b) ;
(b) mélanger un composé métallo-organique ou un mélange de composés métallo-organiques qui contient des ligands hydrolysables afin de favoriser une réaction d'hydrolyse ;
(c) solidifier le mélange via une transition sol-gel, ce par quoi on prépare un gel qui possède des domaines phasiques interconnectés tridimensionnels, l'un riche en solvant, l'autre riche en composant inorganique où les pores de surface sont contenus ;
(d) désintégrer le gel en particules ;
(e) libérer l'agent de dissolution de matrice à partir de son précurseur, ce par quoi l'agent de dissolution de matrice modifie la structure dudit composant inorganique ;
(f) éliminer le solvant par séchage par évaporation et/ou traitement thermique ;

(g) revêtir le matériau obtenu dans l'étape (f) par un polymère afin de fournir des propriétés de surface hydrophobes.

9. Procédé selon la revendication 8, **caractérisé en ce que** l'étape (g) est effectuée par la réaction du matériau obtenu dans l'étape (f) avec un composé de silane.

10. Procédé selon la revendication 8 et/ou 9, **caractérisé en ce que** le matériau particulaire obtenu dans l'étape (f) est calciné préalablement à l'exécution de l'étape (g).

11. Matériau à base d'oxyde de silicium composite, **caractérisé en ce que** le matériau à base d'oxyde de silicium composite se trouve sous la forme de particules et est constitué d'un matériau à base d'oxyde de silicium essentiellement amorphe, qui comprend des macropores et des mésopores et qui est modifié par réaction avec un composé de silane afin de fournir des propriétés de surface hydrophobes, où les macropores possèdent un diamètre moyen > 0,1 $\mu$m et les mésopores possèdent un diamètre moyen allant de 2 à 100 nm.

12. Matériau à base d'oxyde de silicium composite selon la revendication 11, **caractérisé en ce que** les particules possèdent une forme irrégulière non sphérique.

13. Système d'administration à libération modifiée, comprenant un agent biologiquement actif et un matériau à base d'oxyde de silicium composite selon la revendication 11 et/ou 12.

14. Système d'administration à libération modifiée selon la revendication 13, **caractérisé en ce que** l'agent biologiquement actif est une substance pharmaceutique.

15. Système à libération modifiée selon la revendication 13 et/ou 14, **caractérisé en ce que** l'agent biologiquement actif possède une solubilité dans l'eau < environ 10 mg/ml, préférablement allant d'environ 0,0001 mg/ml à environ 5 mg/ml et plus préférablement < 1 mg/ml.

16. Système à libération modifiée selon l'une ou plusieurs parmi les revendications 13 à 15, **caractérisé en ce que** l'agent biologiquement actif est présent selon une quantité allant d'environ 0,1 à environ 90% en poids, préférablement d'environ 0,2 à environ 75% en poids, plus préférablement d'environ 5 à environ 40% en poids, tout préférablement d'environ 10 à environ 30% en poids.

Figure 1

Figure 2

Figure 3

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6207098 B1

- US 7648761 B2

### Non-patent literature cited in the description

- **D.Y. ZHAO et al.** Triblock copolymer syntheses of mesoporous silica with periodic 50 to 300 angstrom pores. *Science,* 1998, vol. 279, 548-552
- **J.S. BECK et al.** A new family of mesoporous molecular sieves prepared with liquid-crystal templates. *J. Am. Chem. Soc.,* 1992, vol. 114, 10834-10843
- **M. VALLET-REGI et al.** A new property of MCM-41: drug delivery system. *Chem. Mater.,* 2001, vol. 13, 308-311
- **P. HORCAJADA et al.** Influence of pore size of MCM-41 matrices on drug delivery rate. *Microporous Mesoporous Mater,* 2004, vol. 68, 105-109
- **J. ANDERSSON et al.** Influences of material characteristics on ibuprofen drug loading and release profiles from ordered micro- and mesoporous silica matrices. *Chem. Mater,* 2004, vol. 16, 4160-4167
- **B. MUNOZ et al.** MCM-41 organic modification as drug delivery rate regulator. *Chem. Mater.,* 2003, vol. 15, 500-503
- **A. L. DOADRIO et al.** A rational explanation of the vancomycin release from SBA-15 and its derivatives by molecular modeling. *Microporous Mesoporous Mater.,* 2010, vol. 132, 559-566
- **A. L. DOADRIO et al.** functionalized of mesoporous materials with long and can change as strategy for controlling drug delivery pattern. *J. Mater. Chem.,* 2006, vol. 16, 462-466
- **I. IZQUIERDO-BARBA et al.** Release evaluation of drugs from ordered three-dimensional silica structures. *Eur J Pharm Sci,* 2006, vol. 26, 365-373

- **F. QU et al.** Effective controlled release of captopril by silylation of mesoporous of MCM-41. *ChemPhysChem.,* 2006, vol. 7, 400-406
- **A. BÖGERSHAUSEN et al.** Drug release from self-assembled inorganic-organic hybrid gels and gated porosity detected by positron annihilation lifetime spectroscopy. *Chem. Mater.,* 2006, vol. 18, 664-672
- **Z.G. SHI et al.** Synthesis and characterization of hierarchically porous silica microspheres with penetrable macropores and tunable mesopores. *Micropor. Mesopor. Mater.,* 2008, vol. 116, 701-704
- **S. PETIT ; G. COQUEREL.** *The amorphous state,* 259-286
- **R. HILFIKER.** Polymorphism in the Pharmaceutical Industry. Wiley-VCH, 2006
- **DVS, S. M. REUTZEL-EDENS ; A.W. NEWMAN.** *Physical Characterization of Hygroscopicity in Pharmaceutical Solids,* 235-258
- **E. CALLERI et al.** Evaluation of a monolithic epoxy silica support for penicillin G acylase immobilization. *Journal of Chromatography A,* 2004, vol. 1031, 93-100
- **G. AMIDON et al.** *Pharm. Res.,* 1995, vol. 12, 413-420
- Handbuch der HPLC [Handbook of HPLC. GIT-Verlag, 1989
- **K. K. UNGER.** Porous Silica. Elsevier Scientific Publishing Company, 1979
- **S. JAMZAD et al.** *AAPS PharmSciTech,* 2006, vol. 7, E1-E6